# EUROPEAN PATENT APPLICATION

(11) **EP 4 548 879 A2**
(43) Date of publication of application: **07.05.2025**
(21) Application number: 25164464.7
(22) Date of filing: 08.05.2020
(51) Int. Cl.: A61F 2/24

(54) **CONTINUOUS TETHERED TISSUE ANCHOR AND ASSOCIATED SYSTEMS AND METHODS**

(30) Priority: 09.05.2019 US 201962845666 P; 24.01.2020 US 202062965595 P
(62) Divisional of application: 20728634.5
(71) Applicant: W. L. Gore & Associates, Inc., Newark, DE 19711 (US)
(72) Inventor: YOUNG, Patrick S., Newark, 19711 (US)
(74) Representative: HGF

(57) **Abstract**

The invention relates to tissue anchor in medical procedures. More specifically the invention relates to a tethered anchor. The tethered anchor includes a tether portion that is elongate and extends between a first end and a second end, the tether portion being formed of a flat film construct; and an anchor portion that defines a width that is greater than a width of the tether portion, the anchor portion being integrally formed with the tether portion of the flat film construct and extending continuously from the second end of the tether portion, the anchor portion having a first aperture through which the tether portion extends.

## Description

### CROSS-REFERENCE TO RELATED APPLICATION

This application claims the benefit of Provisional Application No. 62/965,595, filed January 24, 2020, and also claims the benefit of Provisional Application No. 62/845,666, filed May 9, 2019, both of which are incorporated herein by reference in their entireties for all purposes.

### BACKGROUND

Various tissue anchors have been proposed for delivery into a body of a patient. In some examples, such anchors are delivered using a percutaneous or transcatheter approach (e.g., using a delivery catheter). Some tissue anchors have been proposed that utilize a pledget and tether arrangement. Such anchors may be utilized to secure anatomical structures to one another, to secure an anatomical structure to an implanted device, or to secure two implanted devices together, for example.

### SUMMARY

Various examples relate to tissue anchors, methods used to form tissue anchors, and associated methods of forming tissue anchors. Various disclosed concepts relate to continuous, or integral implantable tissue anchors including an anchor portion and a tether portion that are seamlessly interconnected. Some examples relate to a tether portion and anchor portion formed from a film construct. In some implementations, the tether and anchor portions are confirmed continuously as part of a cutting procedure for a tubular member maintained on a mandrel.

According to one example ("Example 1"), a tethered anchor includes a tether portion that is elongate and extends between a first end and a second end, the tether portion being formed of a flat film construct and an anchor portion that defines a width that is greater than a width of the tether portion. The anchor portion is integrally formed with the tether portion of the flat film construct and extends continuously from the second end of the tether portion. The anchor portion has a first aperture through which the tether portion extends.

According to another example further to Example 1 ("Example 2"), the flat film construct comprises a plurality of layers of film material.

According to another example further to Example 1 ("Example 3"), the tethered anchor further includes at least one radiopaque marker inserted between the plurality of layers of film material.

According to another example further to Example 1 ("Example 4"), the tether portion and the anchor portion have substantially the same thickness where the tether portion extends from the anchor portion.

According to another example ("Example 5"), a tethered anchor is prepared by a process of cutting a preform into a tether portion and an anchor portion, the tether portion extending continuously from the anchor portion, forming a first aperture in the anchor portion, and passing a length of the tether portion through the first aperture in the anchor portion.

According to another example further to Example 5 ("Example 6"), cutting the preform into the tether portion and the anchor portion includes defining a continuous and integral transition between the tether portion and the anchor.

According to another example further to Example 5 ("Example 7"), cutting the preform into the tether portion and the anchor portion includes forming the anchor portion with a greater width than the tether portion.

According to another example further to Example 5 ("Example 8"), the process further includes forming a plurality of apertures in the anchor portion and passing the tether portion through each of the plurality of apertures, such that the anchor portion defines one or more pleats upon tensioning the tether portion.

According to another example further to Example 5 ("Example 9"), the process further includes forming the preform by wrapping one or more layers of material onto a mandrel.

According to another example further to Example 9 ("Example 10"), the process further includes inserting at least one radiopaque marker between layers of material.

According to another example ("Example 11"), a tethered anchor is formed by cutting a preform into a tether portion and an anchor portion, the tether portion extending continuously from the anchor portion, forming a first aperture in the anchor portion, and passing a length of the tether portion through the first aperture in the anchor portion.

According to another example further to Example 11 ("Example 12"), cutting the preform into the tether portions and the anchor portion includes defining a continuous and integral transition between the tether portion and the anchor.

According to another example further to Example 11 ("Example 13"), cutting the preform into the tether portions and the anchor portion includes forming the anchor portion with a greater width than the tether portion.

According to another example further to Example 11 ("Example 14"), the method of forming the tethered anchor further includes forming a plurality of apertures in the anchor portion and passing the tether portion through each of the plurality of apertures, such that the anchor portion defines one or more pleats upon tensioning the tether portion.

According to another example further to Example 11 ("Example 15"), the method of forming the tethered anchor further includes forming the preform by wrapping one or more layers of material onto a mandrel.

According to another example further to Example 15 ("Example 16"), the method of forming the tethered anchor further includes inserting at least one radiopaque marker between the layers of material.

According to one example ("Example 17"), a method of treating heart valve disfunction includes arranging a tethered anchor at a target location within a patient, the tethered anchor including a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends.

According to another example further to Example 18 ("Example 17"), the method of treating heart valve disfunction includes a tethered anchor that is configured for chordal repair or replacement or treating a defective valve.

According to one example ("Example 19"), a tethered anchor includes a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends; and at least one needle coupled to the tether portion.

According to another example further to Example 19 ("Example 20"), the tethered anchor also includes at least one needle includes a first needle coupled to a first end of the tether portion and a second needle coupled to a second end of the tether portion.

According to one example ("Example 21"), a tethered anchor includes a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends; and at least one tissue anchor coupled to the tether portion.

According to another example further to Example 21 ("Example 22") the tethered anchor also includes at least one tissue anchor includes a first tissue anchor coupled to a fist end of the tether portion and a second tissue anchor coupled to a second end of the tether portion.

The foregoing Examples are just that and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows a preform according to some embodiments;
FIG. 2 shows a surface of the preform shown in FIG. 1, according to some embodiments;
FIG. 3 shows a suture in a curled-up configuration according to some embodiments;
FIG. 4 shows a continuously formed pledget anchor and suture according to some embodiments;
FIG. 5 shows the pledget anchor and suture of FIG. 4 in a partially actuated configuration;
FIG. 6 shows the pledget anchor and suture of FIGs. 4 and 5 in a fully actuated configuration;
FIG. 7 shows an end portion of a pledget anchor and suture according to some embodiments; and
FIG. 8 shows a pledget anchor and suture as used in chordal repair procedures according to some embodiments;
FIG. 9 shows a pledget anchor and suture according to some embodiments;
FIG. 9A shows a pledget anchor and suture according to some embodiments;
FIG. 9B shows a pledget anchor and suture with an interlocking mechanism according to some embodiments;
FIG. 10 shows the pledget anchor and suture of FIG. 11 in a folded configuration;
FIG. 11 shows a pair of pledget anchors and sutures, connected by an interlocking mechanism according to some embodiments;
FIG. 12 shows a pledget anchor and suture according to some embodiments;
FIG. 13 shows a pledget anchor and suture according to some embodiments;
FIG. 14 shows the pledget anchor and suture of FIG. 15 in a folded configuration according to some embodiments;
FIG. 15 shows a pledget anchor and suture according to some embodiments;
FIG. 16 shows the pledget anchor and suture of FIG. 17 in a folded configuration according to some embodiments;
FIG. 17 shows a pledget anchor and suture according to some embodiments;
FIG. 18 shows the pledget anchor and suture of FIG. 19 being folded according to some embodiments;
FIG. 19 shows the pledget anchor and suture of FIG. 19 according to some embodiments;
FIG. 20 shows a pledget anchor and suture with a plurality of anchor portions according to some embodiments;
FIG. 21A shows a pledget anchor and suture with a cylindrical stop component according to some embodiments;
FIG. 21B shows a pledget anchor and suture with a washer stopper according to some embodiments.
FIG. 22 shows end portions of a pledget anchor and needles arranged at each of the ends according to some embodiments;
FIG. 23 shows an end portion of a pledget anchor and a tissue anchor arranged at the end portion according to some embodiments; and
FIG. 24 shows an end portion of a pledget anchor and a tissue anchor arranged at the end portion according to some embodiments.

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error or minor adjustments made to optimize performance, for example.

As used herein, "couple" means to join, connect, attach, adhere, affix, or bond, whether directly or indirectly, and whether permanently or temporarily.

### Description of Various Embodiments

Various disclosed concepts relate to continuous, or integral implantable tissue anchors including an anchor portion and a tether portion that are seamlessly interconnected. Some examples relate to an anchor, or pledget portion and tether, or suture portion formed continuously from a film construct (e.g., a tubular film construct).

As described in further detail below, the implantable anchors discussed herein may be used in a variety of medical procedures. For example and in certain instances, the implantable anchors may be used in chordal repair. In addition, the implantable anchors may be used for treating a defective valve (e.g., mitral valve, tricuspid valve). The implantable anchors may be wrapped about a circumference of the heart or valve annulus to ensure closure of a valve that is experiencing regurgitation. In addition, the implantable anchors may be used in valve annuloplasty procedures in a heart or closing an opening or aperture formed in a wall of the heart, such as a ventricular or atrial septal wall defect.

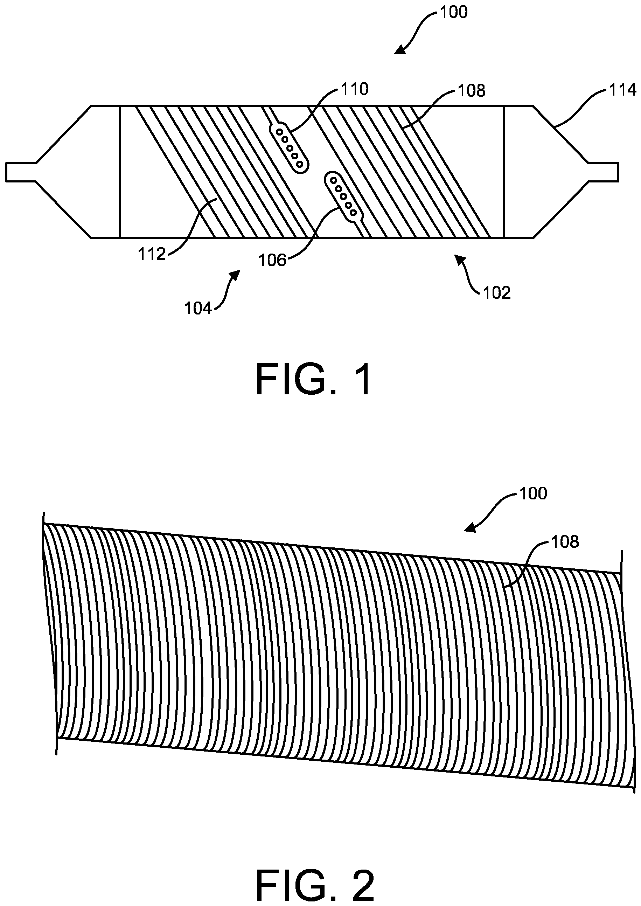

FIG. 1 shows a preform 100 that is cut to form a pair of tethered anchors 102 and 104. The first tethered anchor 102 has an anchor portion 106 and a tether portion 108, and the second tethered anchor 104 also has a corresponding anchor portion 110 and tether portion 112. The preform 100 may be formed by wrapping a film around a mandrel 114 in a helical pattern (e.g., tape-wrapped) or tubular pattern (e.g., cigarette-wrapped), where the mandrel 114 acts as a support structure for the preform 100 during formation thereof. Although wrapping is one means of manufacture, others may be employed as desired, including but not limited to extruding, molding, drawing, or other process.

In some examples, the preform 100 is a film construct made from a plurality of layers of film material (e.g., expanded polytetrafluorethylene, or ePTFE film, or associated composite materials), and the preform 100 is formed by disposing a plurality of layers of the film material onto one another and exposing the layered film material to a predetermined pressure and/or temperature to bond the layers. In one example, a radiopaque marker material is positioned between the layers of film material in any desired configuration or pattern within the preform 100. Although the mandrel 114 may be is tubular, with a circular cross-section, in other examples, the mandrel 114 has a cross-section that is rectangular, ovular, or any other suitable shape, including tapers, steps or other variations in length as desired.

After the preform 100 is prepared, the preform 100 is cut to form one or more tethered anchors, such as the tethered anchors 102 and 104. In one example, the cutting is done using a laser, while in another example, other cutting / forming methods such as water jet cutting, plasma cutting, or mechanical (blade) cutting for example, can be used. Also, although the preform 100 is shown to form two tethered anchors in FIG. 1, other examples can have the preform cut so that one tethered anchor can be formed from a single preform, or three or more tethered anchors can be formed from the preform. For simplicity, moving forward, only the first tethered anchor 102 is mentioned, but it should be understood that the same explanations and descriptions may be applied to the respective portions of the second tethered anchor 104.

The preform 100 is optionally cut such that the tether portion 108 has a consistent width, as shown in FIG. 2, and the anchor portion 106 has a width greater than the width of the tether portion 108. Although a substantially constant width is shown for the respective tether and anchor portions 106, 108, it is to be understood that varying widths and non-linear (e.g., sinuous) cuts are also contemplated. Regardless, the anchor portion 106 is integrally formed with the tether portion 108 of the flat film construct such that the anchor portion 106 extends continuously from one end of the tether portion 108. In one example, the anchor portion 106 and the tether portion 108 as formed have substantially the same thickness since the anchor portion 106 and the tether portion 108 are made of the same homogeneous material. Subsequent to cutting, the tether portion 108 may be reformed from a flat profile to have a rounder profile than the anchor portion 106. As shown, there is a smooth, continuous and integral transition between the anchor portion 106 and the tether portion 108, which may assist in reducing wearing at the transition between the two portions of the first tethered anchor 102.

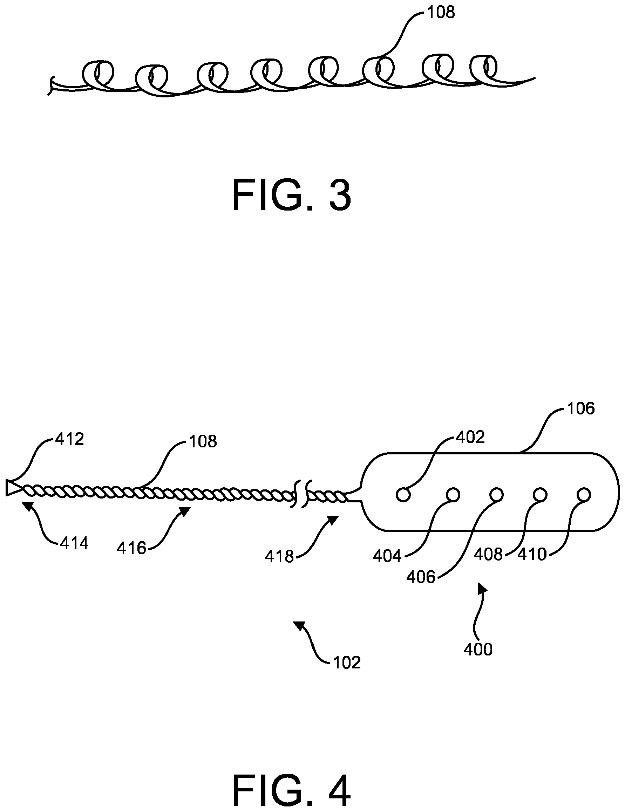

FIG. 3 shows the tether portion 108 in a curled, or twisted configuration (e.g., after being removed from the mandrel 114). The number of turns or twists per unit length may be adjusted as desired. In some examples, the tether portion 108 is twisted and compressed (e.g., in a "candy cane" configuration) by drawing the tether portion 108 through a die configured to twist and / or compress the tether portion 108. The tether portion 108 is optionally bonded, sintered, or otherwise formed into a more circular cross-section due to this twisting and compression process, or the like. For example, the tether portion 108 can be tightly curled in a compressed, twisted configuration so that the cross-section thereof is circular or ovular as indicated generally in FIG. 4. Although this curling / compaction may change the overall profile of the tether portion 108, as well as the density of the tether portion 108, the mass of the tether portion 108 remains unchanged.

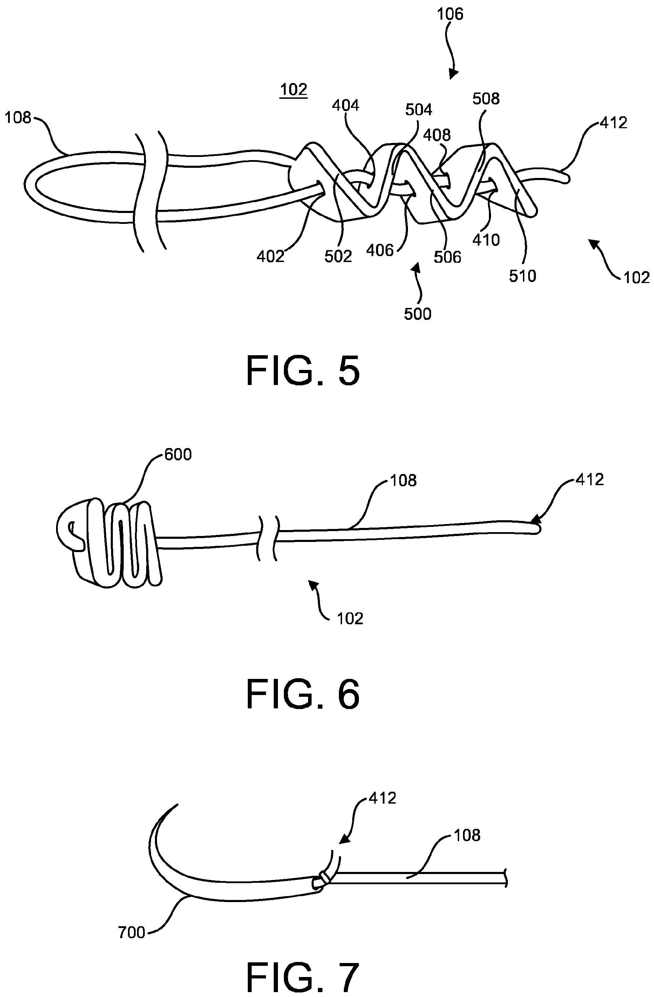

FIG. 4 shows the tethered anchor 102 after the tether portion 108 is twisted and compacted to achieve a more circular cross-section, where the tether portion 108 transitions smoothly into the anchor portion 106 which includes a plurality of crossing apertures 400, such as first through fifth crossing apertures 402, 404, 406, 408, 410 through which the tether portion 108 may pass in an alternating, zig-zag or laced pattern as shown in FIG. 5. The crossing apertures 400 may be pre-formed into the anchor portion 106 during manufacture (e.g., the crossing apertures 400 may be laser-cut when the rest of the tethered anchors 102 and 104 are cut) such that the tether portion 108 only needs to be guided through, extended through, or disposed therein sequentially through the crossing aperture(s) 400 to create an arrangement in which the end portion 412 may be tensioned to collapse the anchor portion 106 to transfer the anchor portion 106 from a delivery configuration to an anchoring configuration. In some examples, the anchor portion 106 is formed without any pre-formed crossing aperture or opening, and the crossing apertures are formed after the anchor portion 106 is formed by puncturing the surface of the anchor portion 106 to provide the openings through which the tether portion 108 can pass through.

Either as part of manufacture, or prior to or during implantation, a needle or other implement can be utilized to deliver end portion 412 through the crossing apertures 400 and / or to form the crossing apertures 400. The size of the crossing apertures 400 can be the same as, smaller than, or larger than the thickness of the tether portion 108. In some examples, the crossing apertures 400 are smaller than the thickness of the tether portion 108, but the material of the anchor portion 106 is expandable or elastic such that the tether portion 108 can pass through the crossing apertures without causing damage to either the tether portion 108 or the anchor portion 106. The crossing apertures 400 can be evenly spaced apart or have varied spacing. In one example, the crossing apertures 400 are positioned along a relatively straight line, although the crossing apertures 400 may be staggered or otherwise arranged. And, although FIG. 4 shows five crossing apertures, it is understood that any suitable number of crossing apertures can be used.

In some examples, the tether portion defines a proximal section 414 extending from the end portion 412, a distal section 418 adjacent the anchor portion 106, and an intermediate section 416 between the proximal section 414 and the distal section 418. The proximal section 414, intermediate section 416, and the distal section 418 may make up any of a variety of percentages of the length of the tether portion 108, such as 1/3-1/3-1/3, 90%-10%-10%, or any of a variety of combinations. In some examples, each of the sections 414, 416, 418 makes up at least 5% of the length of the tether portion 108.

FIG. 5 shows an intermediate configuration of the tethered anchor 102 where the end portion 412 is inserted through each of the crossing apertures 400 of the anchor portion 106, and the anchor portion 106 is bent or folded to form a plurality of pleats 500. In some examples, and as shown, the number of pleats 500 formed is the same as the number of crossing apertures 400, such as first through fifth crossing apertures 402, 404, 406, 408, 410, on the anchor portion 106. This configuration is achieved when the end portion 412 is brought through the first crossing aperture 402, such that the anchor portion 106 is folded between the first crossing aperture 402 and the second crossing aperture 404 and the end portion 412 passes through the second crossing aperture 404. These steps are repeated until end portion 412 passes through all the crossing apertures, as shown in FIG. 5, to form a plurality of pleats 500 in the anchor portion 106, e.g., a first pleat 502, a second pleat 504, a third pleat 506, a fourth pleat 508, and a fifth pleat 510. The pleats 500 resemble a zigzag, or accordion shape when seen from the side. This arrangement allows the anchor portion 106 to initially take on an elongate, more linear profile and then upon tensioning the tether portion 108 the anchor portion 106 folds down to an enlarged, transverse profile relative to the longitudinal axis. In this manner the anchor portion 106 can be initially deployed in a lower profile, or delivery configuration (e.g., by being inserted through a tissue structure) and subsequently tensioned down to an enlarged profile, or anchoring configuration.

FIG. 6 shows the final product in an enlarged profile or anchoring configuration after the end portion 412 is pulled away from the anchor portion 106. As shown, the pleats 500 are collapsed onto one another after tensioning the tether portion 108 to form an anchor 600 with a greater thickness than that of the anchor portion 106 in its original form shown in FIG. 4.

FIG. 7 shows one embodiment of the end portion 412 of the first tether portion 108 as previously mentioned. In this embodiment, the end portion 412 has a needle 700 coupled to (*e.g.*, tied onto, adhered) the end portion 412 such that the needle 700 is implemented as part of the tether portion 108. In some examples, the needle 700 can be used for skin closure, suturing soft tissue with minimal trauma, or other microsurgical procedures. The needle 700 may have a pointed edge that pierces the tissue, and in some examples also may include a cutting blade edge that can cut open tissue during microsurgical procedures. The needle 700 may have a generally C-shaped, J-shaped, or S-shaped configuration according to some examples. Additionally, the needle 700 can be of any suitable shape (straight, curved, hooked, bent, twisted, etc. in some examples), size (shorter than 3 mm, between 3 mm and 5 mm, between 5 mm and 7 mm, or longer than 7 mm in length in some examples), and material (nitinol, stainless steel, or other types of metal in some examples) as appropriate for the surgical procedure.

One example of an application of the tethered anchor 102 as described herein is chordal repair, such as with respect to the valve chords of the heart. When valve chords are broken and the valve leaflets cannot sufficiently open and close to control the flow of blood therethrough, a tethered anchor as shown herein can be used to support the opening and closing of the valve leaflets by attaching the anchor portion to the valve leaflets and the other end of the tether portion to the papillary muscle, creating an artificial valve chord. As the papillary muscle contracts, the tether portion pulls the anchor portion, causing the leaflets to open and allow blood flow therethrough. In this example, the anchor portion is attached on the side of the leaflets opposite from the papillary muscle, so that when the papillary muscle contracts, the anchor portion prevents the tethered anchor from being pulled through an aperture through which the tether portion of the tethered anchor passes.

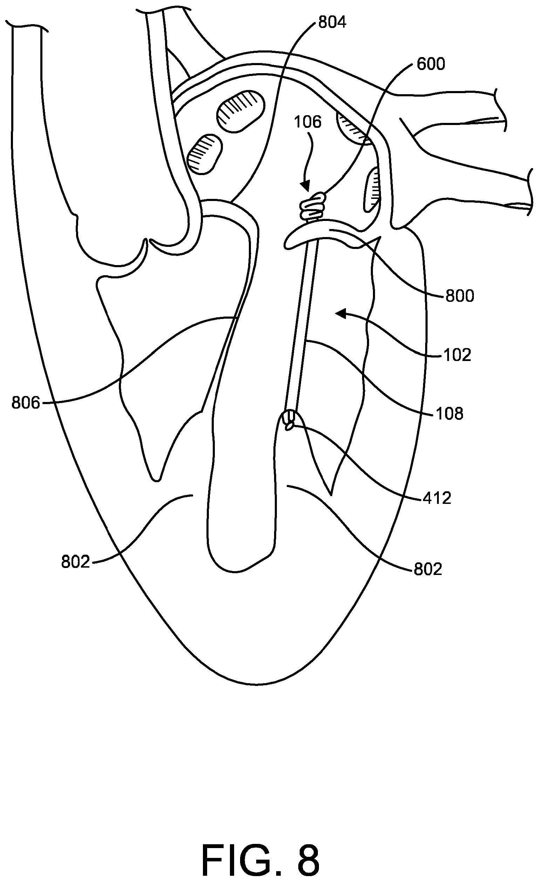

FIG. 8 shows one example of this application. In a healthy valve, a set of structures called chordae tendinea 806 connect each leaflet 804 to papillary muscle 802. However, when the chordae tendinea are torn or ruptured, the tethered anchor 102 is attached to one or more of the valve leaflets 800 whose chordae tendinea are torn by first placing or disposing the anchor portion 106 against a distal surface of the valve leaflet 800 with respect to a papillary muscle 802. Then, the anchor portion 106 is bent or folded over itself to form the plurality of pleats 500 upon tensioning of the tether portion 108 as shown in FIG. 5. The tether portion 108 is passed through each of the crossing apertures 400 in a zigzag pattern, after which the tether portion 108 is tensioned to fully collapse the pleats 500 to form the anchor 600. The end portion 412 of the tether portion 108 is subsequently attached to the papillary muscle 802 so the movement of the papillary muscle 802 induces the movement of the valve leaflet 800.

Another example of an application of the tethered anchor 102 is in valve annuloplasty procedures in a heart, for example, where a ring around the valve in the heart (annulus) widens and changes from its normal shape. A tethered anchor 102 may be arranged to tighten or reinforce the annulus of the valve. This may prevent leakage of blood through the widened valve. The tethered anchor as described herein can be used such that the annuloplasty devices remain secured to the annulus and continue to assist in restoring the normal function of the valve.

Another example use of the tethered anchor 102 an application is in closing an opening or aperture formed in a wall of the heart, such as a ventricular or atrial septal wall defect. The tethered anchor 102 can be used to help close the opening from within the heart at the inner side of the heart wall, such that the flow of blood through the heart does not cause the anchor to detach from the wall through prolonged use due to the constant pressure exerted from within the heart.

In some examples, echogenicity is a factor to be considered when implementing the tethered anchor such that the anchor can be accurately captured during medical imaging such as medical ultrasonography. For example, a material is more echogenic if there is hyperechoic air implemented into the material, and the material is more capable of capturing such hyperechoic air if the material comprises a hydrophobic water-immiscible matrix. In one example, the tethered anchor is made of a hydrophobic material and/or is coated with a layer of hydrophobic agent to prevent the hyperechoic air from escaping to the environment. In another example, the tethered anchor has radiopaque fillers such as tungsten powder with a small particle size (such as less than 1 micron) such that the radiopaque fillers do not interfere with the function of the tethered anchor but allows for the tethered anchor to be visible under fluoroscopy or X-ray.

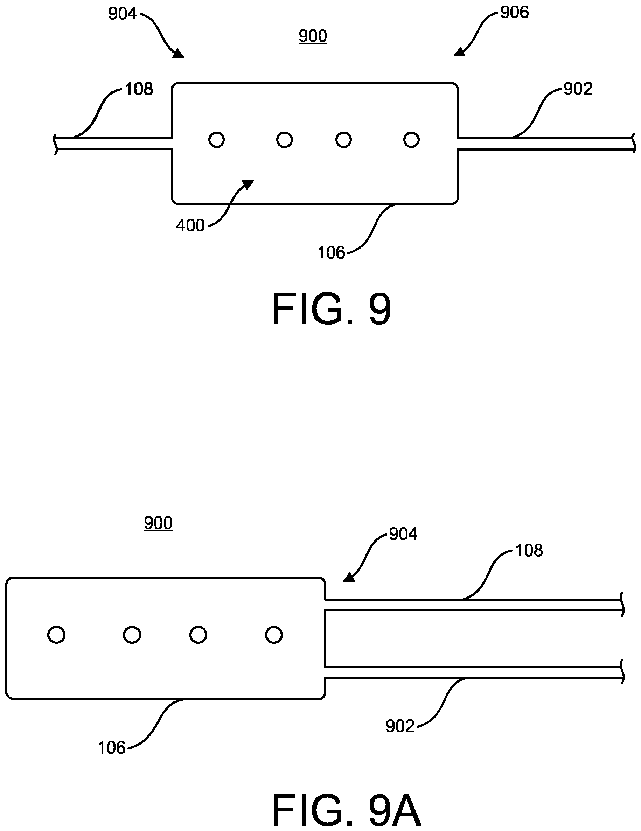

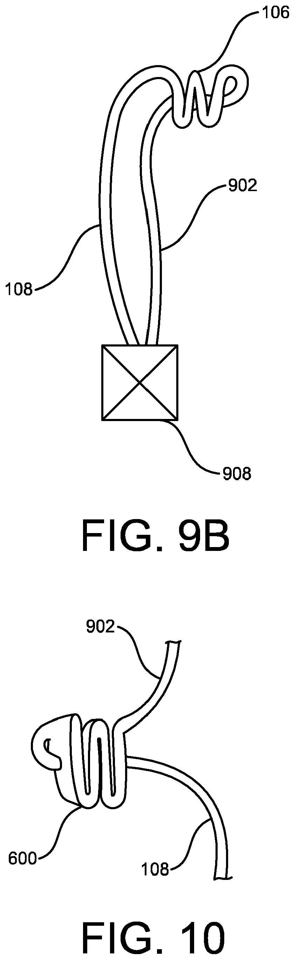

FIGs. 9 and 10 show a tethered anchor 900 with a first tether portion 108, or suture, a second tether portion 902, and an anchor portion 106, or pledget, with a plurality of crossing apertures 400, in accordance with an embodiment. The anchor portion 106 has two ends, a first end 904 and a second 906 opposite to the first end 904. The first tether portion 108 extends from the first end 904 and the second tether portion 902 extends from the second end 906 such that, when the anchor 600 is formed by passing the first tether portion 108 through the crossing apertures 400 as previously described, the second tether portion 902 extends from the anchor 600 as shown in FIG. 10. In some examples, the second tether portion 902 may be used to attach an additional component to the anchor 600. In some examples, the second tether portion 902 can extend from the same end (904 or 906) as the first tether portion 108, as shown in FIG. 9A. FIG. 9B shows an example of the needle 700 in which the ends of the first tether portion 108 and the second tether portion 902 are interlocked using an interlocking mechanism 908 to form a parachute-like configuration. In some examples, a first needle (not shown) may be attached to a free end of the first tether portion 108 and a second needle (not shown) may be attached to a free end of the second tether portion 902, where the free end is the end of the tether portion that is not attached or connected to the anchor portion 106. These needles may be the needle 700 as explained above and shown in FIG. 7. In some examples, the two needles are different from each other, such as having different shapes, sizes, and/or materials.

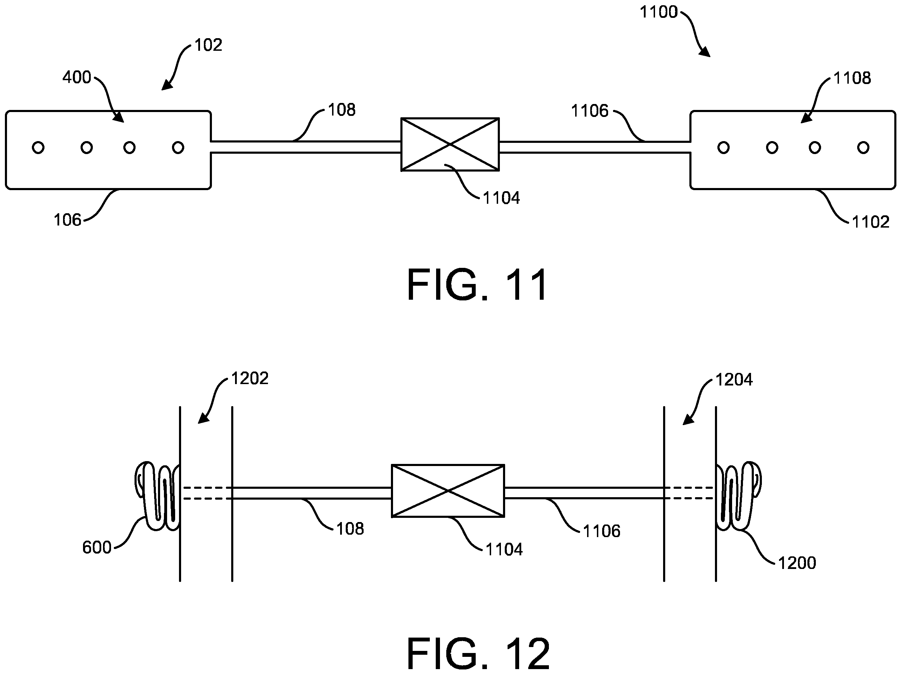

FIGs. 11 and 12 show a pair of tethered anchors 102 and 1100 connected to each other via an interlocking mechanism 1104. The first tethered anchor 102 has the first tether portion 108 extending from the first anchor portion 106 and a second tethered anchor 1100 has the second tether portion 1106 extending from a second anchor portion 1102. The ends of the two tether portions 108 and 1106 can be interlocked using the interlocking mechanism 1104 such that the two tethered anchors 102 and 1100 can essentially be utilized as a single continuous tether with an anchor 600 or 1200 on each end, as shown in FIG. 12.

In this case, the anchors 600 and 1200 are located on two opposing sides of two walls 1202 and 1204, and the interlocking mechanism 1104 is disposed in a space between the walls 1202 and 1204 separated from the two anchors 600 and 1200. The anchors 600 and 1200 are formed before the two tether portions 108 and 1106 become interlocked. Initially, the first anchor 600 is formed by inserting the tether portion 108 through the crossing apertures 400 as previously described, and the second anchor 1200 is similarly formed by inserting the second tether portion 1106 through crossing apertures 1108 of the second anchor portion 1102. Then, the two free ends of the tether portions 108 and 1106 are coupled together using the interlocking mechanism 1104.

In some examples, the interlocking mechanisms 908 and 1104 may be clamps, clips, locks, latches, or any suitable mechanism to secure the two tether portions 108 and 902 (in FIG. 9B) or 1106 (in FIGs. 11 and 12) together. In some examples, the interlocking mechanisms 908 and 1104 also have a tightening capability which can adjust the length of the tether formed by interlocking the two tether portions 108 and 902 or 1106. For example, there may be a screw or knob on the interlocking mechanism 1104 which, when activated, brings the two anchors 600 and 1200 closer together.

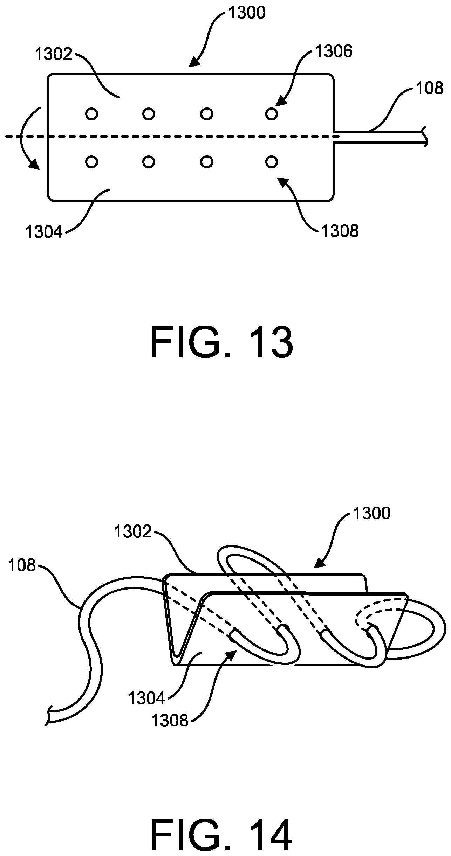

FIGs. 13 and 14 show an anchor portion 1300 with a first row of crossing apertures 1306 located in a first section 1302 of the anchor portion 1300 and a second row of crossing apertures 1308 located in a second section 1304 of the anchor portion 1300, in accordance with an embodiment. The tether portion 108 extends from the anchor portion 1300 at the first section 1302, the second portion 1304, or therebetween. The anchor portion 1300 can be folded along the broken line as shown in FIG. 13. The first row of crossing apertures 1306 align with the second row of crossing apertures 1308 when the anchor portion 1300 is folded. After folding the anchor portion 1300, the tether portion 108 crosses each of the first and second rows of crossing apertures 1306 as shown in FIG. 14 to maintain the orientation of the first and second sections 1302 and 1304 of the anchor portion 1300. Applying a force on the tether portion 108 directed away from the anchor portion 1300 causes the folded anchor portion 1300 to collapse, similar to the example shown in FIG. 2, after which the anchor portion 1300 becomes a folded anchor (not shown).

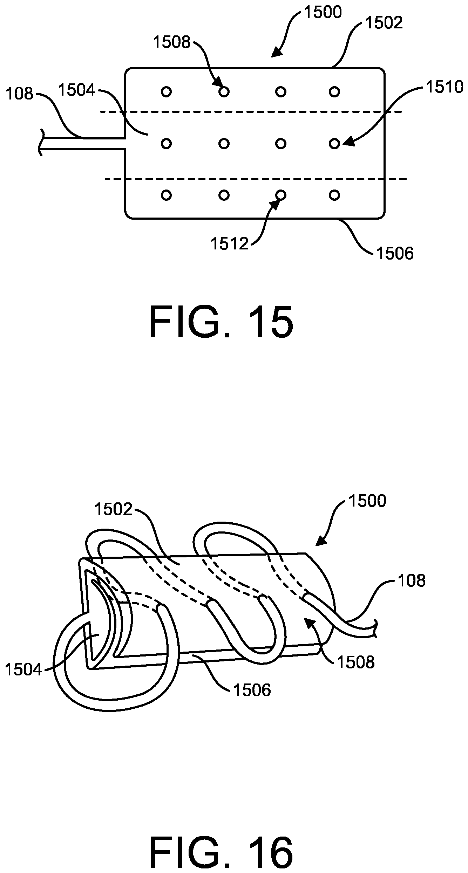

FIGs. 15 and 16 show an anchor portion 1500 with a first row of crossing apertures 1508 located in a first section 1502 of the anchor portion 1500, a second row of crossing apertures 1510 located in a second section 1504 of the anchor portion 1500, and a third row of crossing apertures 1512 located on a third section 1506 of the anchor portion 1500, in accordance with an embodiment. Similar to the anchor portion 1300, the anchor portion 1500 is foldable along the broken lines, where the first, second, and third rows of crossing apertures 1508, 1510, 1512 overlap with each other in the folded configuration, during which the tether portion 108, which may extend from any one of the three sections 1502, 1504, 1506 or therebetween. FIG. 16 shows the anchor portion 1500 being folded in a trifold or "letter-fold" configuration (e.g., resembling how a letter would be folded prior to being placed inside an envelope). Applying a force on the tether portion 108 as previously explained collapses the folded anchor portion 1500 into a folded anchor (not shown). It should be understood that any other number of sections (for example, four or greater), may be used as appropriate, depending on the thickness of the anchor portion that is being folded to form the anchor.

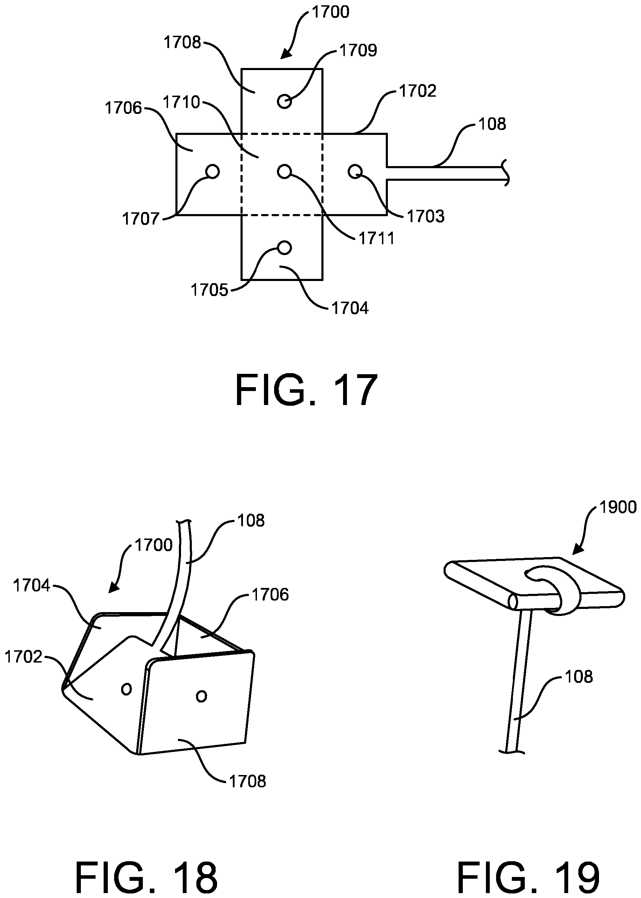

FIGs. 17 to 19 show a cross-shaped anchor portion 1700 with five sections or pleats 1702, 1704, 1706, 1708, 1710 and five crossing apertures 1703, 1705, 1707, 1709, 1711, respectively, located at or proximate the center of each section. In some examples, each pleat 1702, 1704, 1706, 1708, 1710 may be substantially square in shape, with each side of the outer periphery of the cross-shaped anchor portion 1700 being equal or similar in length. The tether portion 108 may extend from any one of the five pleats 1702, 1704, 1706, 1708, 1710. FIG. 18 shows an example of how the anchor portion 1700 can be folded along the broken lines shown in FIG. 17, and FIG. 19 shows an anchor portion 1700 that results from folding the anchor portion 1700 and having the tether portion 108 pass through the crossing apertures 1703, 1705, 1707, 1709, 1711 which align with each other in the folded configuration. Advantages in increasing the number of foldable elements as shown in FIGs. 13 to 19 include being able to adjust the thickness of the anchor that results from folding the anchor portion(s) and passing a tether therethrough. The increased thickness can also increase the stability and durability of the anchors in some examples.

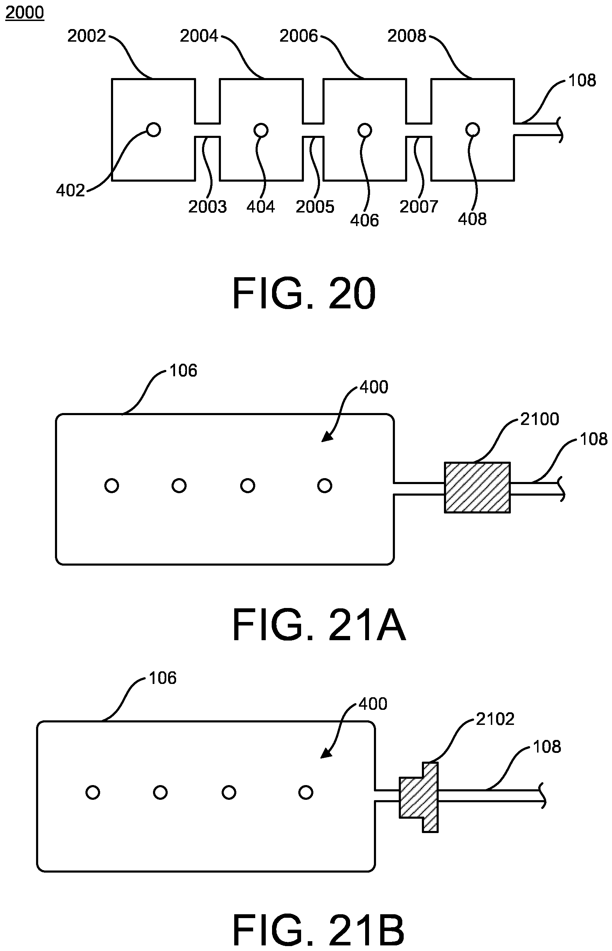

FIG. 20 shows a tethered anchor 2000 according to another embodiment which includes a plurality of anchor portions 2002, 2004, 2006, and 2008. Although FIG. 20 shows four anchor portions, any suitable number of anchor portions can be implemented in other examples. Each pair of neighboring anchor portions is connected with a connecting member such that anchor portions 2002 and 2004 are connected with a connecting member 2003, anchor portions 2004 and 2006 are connected with a connecting member 2005 and anchor portions 2006 and 2008 are connected with a connecting member 2007. Similar to the previously mentioned embodiments, the anchor portions 2002, 2004, 2006, and 2008 fold onto one another to form an anchor similar to the anchor 600 in FIG. 6.

FIGs. 21A and 21B show two configurations of a stop component coupled to the tether portion 108 to prevent the tether portion 108 from passing through the anchor portion 106 or the crossing apertures 400 located in the anchor portion 106. In FIG. 21A, a stop component 2100 may be coupled to the tether portion 108 or at a junction between the anchor portion 106 and the tether portion 108. The stop component 2100 may take the form of a knot, a swaged structure, a crimped structure, or any other suitable structure that limits the ability of the tether portion 108 to pass through the anchor portion 106 or the crossing apertures 400. For example, the diameter of at least a portion of the stop component 2100 can be wider than the crossing apertures 400. In FIG. 21B, a swaged stopper 2102 that has a T-shaped cross section may be coupled to the tether portion 108. The stop component 2100 and the swaged stopper 2102 may have any suitable shape and cross section, and be composed of any suitable material such as metal, rubber, silicone, as well as other elastic or plastic polymers, for example.

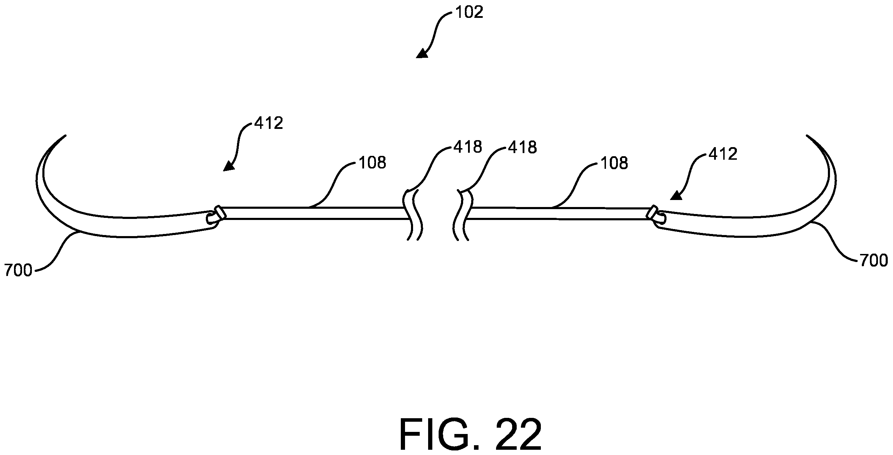

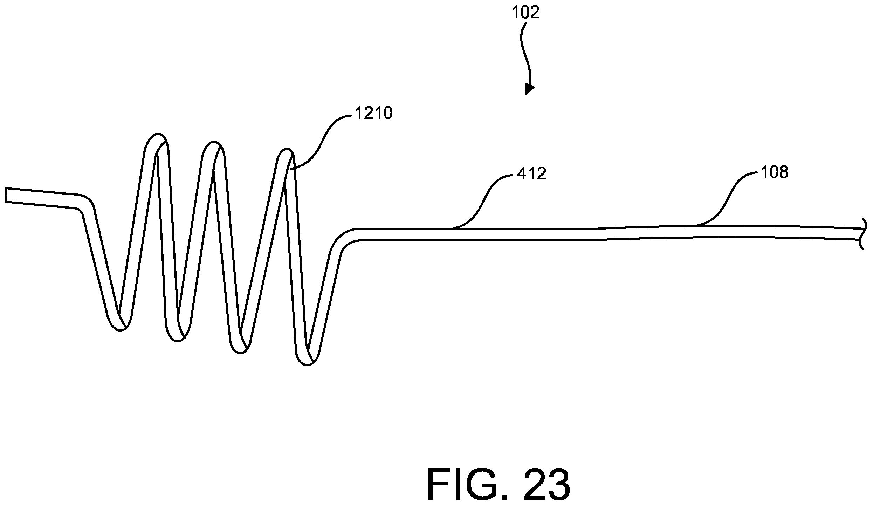

FIG. 22 shows end portions of a tethered anchor 102 and needles 700 arranged at each of the ends according to some embodiments. As shown, each of the end portions 412 of the tethered anchor 102 include needles 700 coupled to the end portion 412 of tether portion 108 the tethered anchor 102. A tether portion 108, as described in detail above, may be attached or coupled to distal portion 418 of the tether portion 108. The dual needled tethered anchor 102 can be used in numerous procedures such as skin closure, suturing soft tissue with minimal trauma, or other microsurgical procedures, cardiac valve repair, or other similar procedures. In certain instances, one or both of the needles may be replaced with a tissue anchor as shown and described with reference to FIGS. 23-24.

FIG. 23 shows an end portion of a tethered anchor 102 and a tissue anchor 1210 arranged at the end portion according to some embodiments. As shown, the tissue anchor 1210 is coupled to an end portion 412 of the tethered anchor 102. The tissue anchor 1210 may be coupled to the end portion 412 of the tether portion 108 such that the tissue anchor 1210 is implemented into the end portion 412 as part of the tethered anchor 102. In certain instances, the tissue anchor 1210 is attached, or adhered to the end portion 412. As shown in FIG. 22, the tissue anchor 1210 may be coupled to both ends of a tethered anchor 102. In addition, more than one tissue anchor 1210 may be arranged with the both ends of a tethered anchor 102 or along the tethered anchor 102.

As shown in FIG. 23, the tissue anchor 1210 includes a helical shape. The tissue anchor 1210 may have one or more coils, as is shown. The number of turns or coils of the tissue anchor 1210 can be varied in order to lengthen or shorten the depth at which the tissue anchor 1210 may be arranged within a leaflet or tissue. The tissue anchor 1210 may be coupled to one or both ends of the tether portion 108 as discussed in detailed above with reference to FIG. 22.

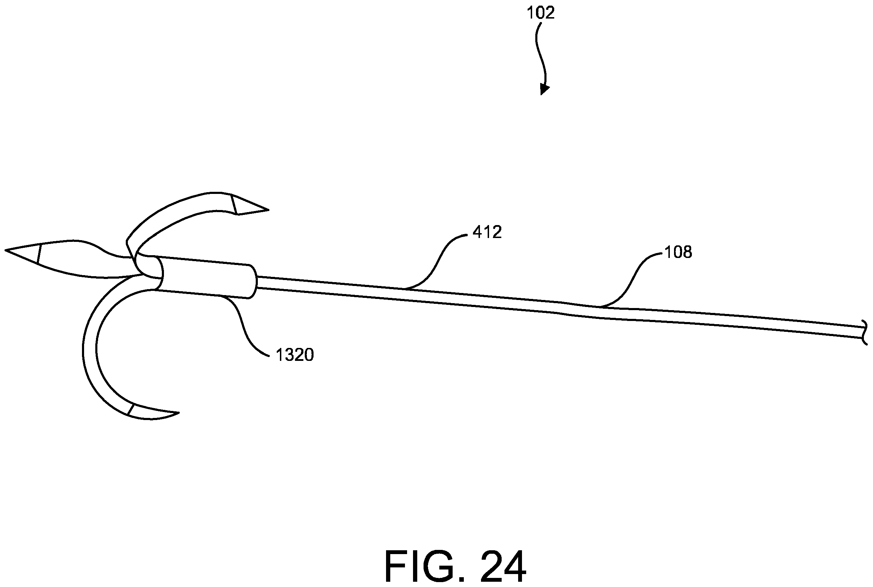

FIG. 24 shows an end portion of a tethered anchor 102 and a tissue anchor 1320 arranged at the end portion according to some embodiments. As shown, the tissue anchor 1320 is coupled to an end portion 412 of the tethered anchor 102. The tissue anchor 1320 may be threaded onto or adhered to the end portion 412 of the tether portion 108 such that the tissue anchor 1320 is implemented into the end portion 412 as part of the tethered anchor 102. As shown in FIG. 22, the tissue anchor 1320 may be coupled to both ends of a tethered anchor 102.

As shown in FIG. 24, the tissue anchor 1320 includes multiple barbs that are configured to embed within tissue. The tissue anchor 1320 may include three, four, five, six, or any additional number of barbs to facilitate anchoring within tissue. The tissue anchor 1320 may be coupled to one or both ends of the tether portion 108 as discussed in detailed above with reference to FIG. 22. In addition, more than one tissue anchor 1320 may be arranged with the both ends of a tethered anchor 100 or along the tethered anchor 100.

According to various examples, the material of the anchored tether may include a fluoropolymer, including without limitation, polytetrafluoroethylene (PTFE) and/or expanded polytetrafluoroethylene (ePTFE), nylon, polypropylene, polyester, PVDF, silk, or other similar materials. In some examples, the anchored tether comprise a membrane, such as ePTFE, that is combined with an elastomer or elastomeric material, such as a fluoroelastomer, to form a composite material, as disclosed herein. It will be appreciated that while various examples are discussed with regard to anchored tether, the various examples and embodiments discussed herein may be universally applied across each of the anchored tethers and/or the various components of the anchored tethers discussed herein.
Features of the invention are set forth in the following Statements:
1. A tethered anchor comprising:
   a tether portion that is elongate and extends between a first end and a second end, the tether portion being formed of a flat film construct; and
   an anchor portion that defines a width that is greater than a width of the tether portion, the anchor portion being integrally formed with the tether portion of the flat film construct and extending continuously from the second end of the tether portion, the anchor portion having a first aperture through which the tether portion extends.
2. The tethered anchor of Statement 1, wherein the flat film construct comprises a plurality of layers of film material.
3. The tethered anchor of Statement 2, further comprising at least one radiopaque marker inserted between the plurality of layers of film material.
4. The tethered anchor of Statement 1, wherein the tether portion and the anchor portion have substantially the same thickness where the tether portion extends from the anchor portion.
5. A tethered anchor prepared by a process comprising:
   cutting a preform into a tether portion and an anchor portion, the tether portion extending continuously from the anchor portion;
   forming a first aperture in the anchor portion; and
   passing a length of the tether portion through the first aperture in the anchor portion.
6. The process of Statement 5, wherein cutting the preform into the tether portion and the anchor portion includes defining a continuous and integral transition between the tether portion and the anchor portion.
7. The process of Statement 5, wherein cutting the preform into the tether portion and the anchor portion includes forming the anchor portion with a greater width than the tether portion.
8. The process of Statement 5, further comprising forming a plurality of apertures in the anchor portion and passing the tether portion through each of the plurality of apertures, such that the anchor portion defines one or more pleats upon tensioning the tether portion.
9. The process of Statement 5, further comprising forming the preform by wrapping one or more layers of material onto a mandrel.
10. The process of Statement 9, further comprising inserting at least one radiopaque marker between layers of material.
11. A method of forming a tethered anchor, the method comprising:
   cutting a preform into a tether portion and an anchor portion, the tether portion extending continuously from the anchor portion;
   forming a first aperture in the anchor portion; and
   passing a length of the tether portion through the first aperture in the anchor portion.
12. The method of Statement 11, wherein cutting the preform into the tether portions and the anchor portion includes defining a continuous and integral transition between the tether portion and the anchor.
13. The method of Statement 11, wherein cutting the preform into the tether portions and the anchor portion includes forming the anchor portion with a greater width than the tether portion.
14. The method of Statement 11, further comprising forming a plurality of apertures in the anchor portion and passing the tether portion through each of the plurality of apertures, such that the anchor portion defines one or more pleats upon tensioning the tether portion.
15. The method of Statement 11, further comprising forming the preform by wrapping one or more layers of material onto a mandrel.
16. The method of Statement 13, further comprising inserting at least one radiopaque marker between the layers of material.
17. A method of treating heart valve disfunction, the method comprising:
   arranging a tethered anchor at a target location within a patient, the tethered anchor including a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends.
18. The method of Statement 17, wherein the tethered anchor is configured for chordal repair or replacement or treating a defective valve.
19. A tethered anchor comprising:
   a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends; and
   at least one needle coupled to the tether portion.
20. The tethered anchor of Statement 19, wherein at least one needle includes a first needle coupled to a first end of the tether portion and a second needle coupled to a second end of the tether portion.
21.A tethered anchor comprising:
   a tether portion that is elongate and extends between a first end and a second end, the tether portion being formed of a flat film construct and an anchor portion that defines a width that is greater than a width of the tether portion, the anchor portion being integrally formed with the tether portion of the flat film construct and extending continuously from the second end of the tether portion, the anchor portion having a first aperture through which the tether portion extends; and
   at least one tissue anchor coupled to the tether portion.
22. The tethered anchor of Statement 21, wherein at least one tissue anchor includes a first tissue anchor coupled to a first end of the tether portion and a second tissue anchor coupled to a second end of the tether portion.

Various features have been specifically described in association with some examples and not in association with others. It is not the intent, however, to preclude the combination of features between examples. Instead, such combinations are specifically contemplated and form a part of this disclosure. The inventive concepts of this disclosure have been described both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

## Claims

1. A tissue anchor system comprising:
a first anchor including a first anchor portion configured to anchor to tissue at a first position and a first tether portion extending from the first anchor portion;
a second anchor including a second anchor portion configured to anchor to tissue at a second position and a second tether portion extending from the second anchor portion; and
an interlock mechanism configured to interlock with the first tether portion and the second tether portion.

2. The tissue anchor system of claim 1, wherein the first anchor portion define crossing apertures configured to receive the first tether portion.

3. The tissue anchor system of claim 1, wherein the interlocking mechanism is a clamp.

4. The tissue anchor system of claim 1, wherein the interlocking mechanism is a clip.

5. The tissue anchor system of claim 1, wherein the interlocking mechanism is a lock.

6. The tissue anchor system of claim 1, wherein the interlocking mechanism is a latch.

7. The tissue anchor system of claim 1, wherein the interlocking mechanism includes a tightening mechanism.

8. The tissue anchor system of claim 7, wherein the tightening mechanism includes a screw or knob on the interlocking mechanism which, when activated, is configured to bring the first anchor portion and the second anchor portion closer together

9. The tissue anchor system of claim 1, wherein the first anchor portion defines a width that is greater than a width of the first tether portion.

10. The tissue anchor system of claim 9, wherein the first anchor portion is integrally formed with the first tether portion of the flat film construct and extends from a second end of the first tether portion.

11. The tissue anchor system of claim 10, wherein a transition between the first anchor portion and the first tether portion is a smooth, continuous and integral transition.

12. The tissue anchor system of claim 11, wherein the first anchor portion and the first tether portion have substantially the same thickness.

13. The tissue anchor system of claim 12, wherein the flat film construct comprises a plurality of layers of film material.

14. The tissue anchor system of claim 13, further comprising at least one radiopaque marker inserted between the plurality of layers of film material.
